# EUROPEAN PATENT SPECIFICATION

(11) **EP 2 651 506 B1**
(45) Date of publication and mention of the grant of the patent: **30.09.2015**
(21) Application number: 11848365.0
(22) Date of filing: 09.08.2011
(51) Int. Cl.: A61N 1/34

(54) **HANDHELD EMG STIMULATOR DEVICE WITH ADJUSTABLE SHAFT LENGTH**
TRAGBARE EMG-STIMULATORVORRICHTUNG MIT EINSTELLBARER SCHAFTLÄNGE
DISPOSITIF PORTATIF STIMULATEUR D'ÉLECTROMYOGRAMME À LONGUEUR D'ARBRE AJUSTABLE

(30) Priority: 13.12.2010 US 422614 P
(43) Date of publication of application: 23.10.2013
(73) Proprietor: Neural Pathways, LLC, Ventura, CA 93003 (US)
(72) Inventor: REA, Ryan, M., Ventura, CA 93003 (US)
(74) Representative: Viering, Jentschura & Partner Patent- und Rechtsanwälte
(86) International application number: PCT/US2011/047011
(87) International publication number: WO 2012/082187

(56) References cited:
- WO-A1-2005/009531
- US-A1- 2006 025 702
- US-A1- 2007 239 187
- US-A1- 2008 221 589
- US-A1- 2008 221 589
- US-A1- 2008 319 508
- US-A1- 2010 148 759
- US-A1- 2010 182 027
- US-B1- 6 309 407
- US-B2- 6 471 659
- US-B2- 7 206 641
- US-B2- 7 309 338

## Description

### FIELD

The present disclosure relates to intraoperative neurophysiological monitoring, and more specifically, to adjustable length stimulation probe devices used in intraoperative neurophysiological monitoring procedures.

### BACKGROUND

Electromyography (hereafter "EMG") is used in Intraoperative Neurophysiological Monitoring (hereafter "IONM") as a way to electromechanically detect "at risk" human motor nerves during surgery. Most human muscles contain human motor nerves. When human motor nerves are electrically stimulated or "excited" the muscles that contain those nerves contract. The muscle contractions are an evoked electromyographic (EMG) event commonly known as a Compound Action Potential (hereafter "CAP") amongst the IONM community.

In proactive nerve location procedures, devices known as "stimulation probes" or "stim probes" are often used to stimulate nerve regions in which nerves are believed to be located to determine if they are in fact present. Certain devices include a handle and an elongated shaft having an electrode at its distal end. The electrode is in electrical communication with a source of stimulation energy. As the surgeon contacts various potential nerve locations, stimulation energy is supplied to evoke responses which are then assessed to determine if nerves are present. In nerve integrity monitoring procedures, known nerve locations are stimulated before and after the occurrence of a potential nerve trauma (e.g., a surgery) to determine if nerve integrity has been compromised.

Typical stimulation probes have a fixed length. Thus, probes are supplied in a variety of lengths to accommodate different procedures. If a procedure involves nerve regions that have different degrees of accessibility or which are different distances from the surface of the patient's body, the surgeon may have to employ multiple probes, which can be costly.

IONM service providers typically supply the operating theater with the necessary equipment to perform nerve integrity monitoring or proactive nerve location procedures. Because of the potential need for probes of varying sizes, many IONM service providers employ an "inventory all" inventory protocol to make sure that all potentially needed probes are on hand at all times. This protocol is costly and logistically challenging for many IONM service providers. Those providers who elect not to use an "inventory all" protocol run the risk of lacking a needed stimulation probe and may have to turn down IONM service provider requests because they do not have a particular probe length that is required for a particular IONM modality. For example, if an IONM service request is submitted by a hospital to an IONM service provider to perform monitoring in a pedicle screw surgery, but the IONM service provider lacks a stimulation probe of the length necessary to monitor the case, e.g., approximately 13 cm long, the service provider will have to decline the request.

WO 2005/009531 A1 discloses an apparatus for use with a cannula that includes a rigid rod which is receivable and movable in the cannula throughout a range of operative positions in which inner and outer end portions of the rod extend longitudinally outward from the inner and outer ends of the cannula. The apparatus further includes a contact electrode which is configured to be supported on the inner end portion of the rod for movement with the rigid rod through the cannula.

Accordingly, a need has arisen for a stimulation probe that addresses the foregoing issues.

The present invention provides a handheld nerve stimulating device with an adjustable length as defined in claim 1. Further embodiments of the present invention are described in the dependent claims.

### BRIEF DESCRIPTION OF THE DRAWINGS

FIG. 1A is a side elevational view of a substantially straight, prior art stimulation probe device of a first fixed length;
FIG. 1B is a side elevational view of a substantially straight prior art stimulation probe device of a second fixed length;
FIG. 2 is a side elevational view of a prior art stimulation probe device with an angled shaft of fixed length;
FIG. 3A is a side elevational view of an embodiment of a substantially straight stimulation probe device with an adjustable shaft in a first position relative to the handle;
FIG. 3B is a side elevational view of the stimulation probe device of FIG. 3A with the adjustable shaft in a second position relative to the handle;
FIG. 4 is a cross-sectional view of the stimulation probe device of FIG. 3A taken along the line 4-4;
FIG. 5 is a cross-sectional view of an alternate embodiment of an adjustable length stimulation probe device with a spring-loaded shaft;
FIG. 6 is a close-up cross-sectional view of the distal handle portion of the stimulation probe device of FIG. 4;
FIG. 7 is a close-up cross-sectional view of the distal elongated shaft portion of the stimulation probe device of FIG. 5;
FIG. 8 is an alternate embodiment of the distal handle portion of stimulation probe device of FIG. 4;
FIG. 9 is an alternate embodiment of the distal elongated shaft portion of the stimulation probe device of FIG. 5;
FIG. 10 is a cross-sectional view of an alternate embodiment of a substantially straight-handled stimulation probe device with an adjustable length insulated sheath;
FIG. 11A is a cross-sectional view of an angled-handle stimulation probe device with an adjustable length insulated sheath, with the sheath in a first configuration relative to the handle;
FIG. 11B is a cross-sectional view of the angle-handled stimulation probe device of FIG. 11A with the sheath in a second configuration relative to the handle;
FIG. 12 is a cross-sectional view of an alternate embodiment of a substantially straight stimulation probe device with an adjustable shaft length;
FIG. 13A is a cross-sectional view of middle handle section of the stimulation probe device of FIG. 12;
FIG. 13B is a cross-sectional view of a proximal handle section of the stimulation probe device of FIG. 12;
FIG. 13C is a cross-sectional view of a distal handle section of the stimulation probe device of FIG. 12;
FIG. 13D is cross-sectional view of a conductive shaft stop of the stimulation probe device of FIG. 12;
FIG. 13E is a cross-sectional view of a ball-tip electrode of the stimulation probe device of FIG. 12;
FIG. 14 is a flow chart depicting a first method of using an adjustable length stimulation probe device; and
FIG. 15 is a flow chart depicting a second method of using an adjustable length stimulation probe device.

### DETAILED DESCRIPTION

The present disclosure relates to a stimulation probe device, or more specifically, to a stimulation probe device with an adjustable length. In certain examples, the device includes a handle of fixed length and an elongated shaft that is movable with respect to the handle to vary the distance between a stimulating electrode and the handle.

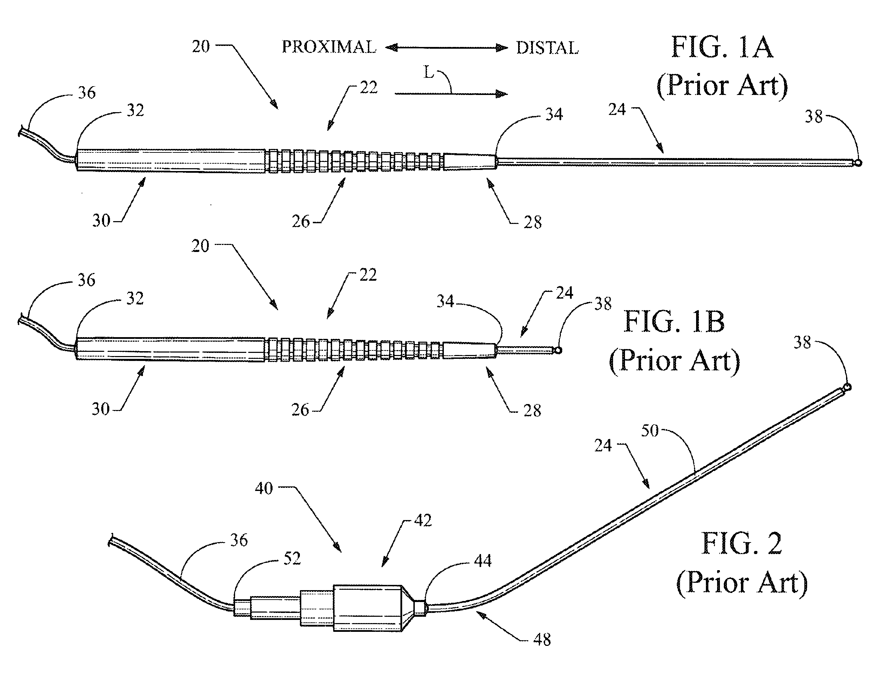

FIGS. 1A and 1B depict two substantially straight prior art stimulation probe devices 20 of fixed length. Each of the stimulation probe devices 20 in the figures comprises the same components. Devices 20 each include a handle 22 and an elongated shaft 24. Handle 22 includes a proximal section 30, a middle section 26, and a distal section 28. Elongated shaft 24 projects distally away from distal handle section 28 in a direction L along the length of the probe device 20. The distal tip 38 of elongated shaft 24 includes an electrode used to contact nerve regions in or on a patient's body to supply stimulation energy used to evoke electrical responses from the patient's nerves.

The devices 20 in FIGS. 1A and 1B each have a fixed overall length as measured from the proximal handle end 32 to the distal end 38. In addition, the lengths of handle 22 and shaft 24 are themselves fixed. Depending on the particular surgical procedure, a surgeon would select one of FIG. 1A or 1B to supply stimulation energy to a nerve region. In certain procedures, both devices 20 in FIGS. 1A and 1B would be required, forcing the surgeon to switch devices 20 mid-procedure.

Electrode 38 is in electrical communication with a source of stimulation energy (not shown) via conductive leads 36 (shown as a single encased wire pair). Elongated shaft 24 may be made of a conductive material itself, or may be non-conductive, with conductive leads disposed internally that place electrode 38 in electrical communication with conductive leads 36. Handles 22 may also include ridges for gripping handle 22.

In certain procedures, the surgeon may need to stimulate nerve regions that cannot be accessed via a straight path into the body. Referring to FIG. 2, a prior art angled stimulation probe device 40 is shown. The device 40 includes a handle 42 and an elongated shaft 24. Elongated shaft 24 includes a proximal section 48 that has a slight curvature and a straight portion 50. Handle 42 includes a distal end 44 and a proximal end 52. Conductive leads 36 project away from the proximal end 52 of handle 42 in the proximal direction. Elongated shaft 24 projects away from handle 42 in the distal direction and in a direction away from the longitudinal axis of handle 42. As with the stimulation probe devices of FIGS. 1A and 1B, electrode 38 is in electrical communication with a source of stimulation energy supplied via conductive leads 36. The overall length of stimulation probe device 40 is fixed, as are the lengths of handle 42 and elongated shaft 24.

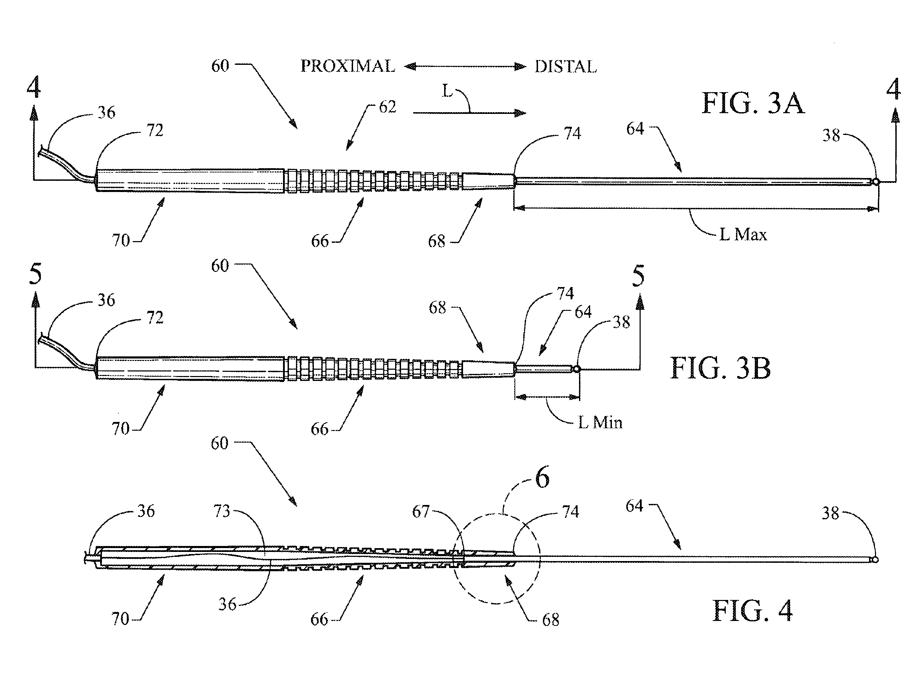

Referring to FIGS. 3A, 3B and 4, a stimulation probe device 60 with an adjustable length is depicted. The overall length of device 60 is adjustable between a maximum length, as shown in FIG. 3A, and a minimum length, as shown in FIG. 3B.

Stimulation probe device 60 includes a handle 62 and an elongated shaft 64. In FIGS. 3A and 3B, handle 62 and elongated shaft 64 are substantially co-axially oriented. However, other configurations may be used, including those in which the longitudinal axes of handle 62 and elongated shaft 64 are spaced apart from one another, as in a parallel configuration.

Handle 62 includes a proximal end 72 and a distal end 74. In the depicted example, handle 62 also includes three sections: proximal section 70, middle section 66, and distal section 68. In the depicted embodiment, the handle sections 70, 66, and 68 are separately formed and then attached to one another. However, in other examples, handle 62 may be integrally formed with one or more of each of the three sections 70, 66, and 68. In certain examples, and as shown in FIGS. 3A and 3B, one or more of the handle sections includes tactile surface features that facilitate gripping the stimulation probe device 60. In the figures, the tactile surface features comprises a plurality of ridges spaced apart along the length of middle handle section 66.

In the depicted embodiment, the elongated shaft 64 is selectively movable in the device length direction (L) with respect to handle 62. The overall length of device 60 is adjusted by adjusting the exposed length of elongated shaft 64 projecting away from distal handle end 74 while the length of handle 62 remains fixed. However, other implementations are possible in which the length of handle 62 is adjusted while the length of elongated shaft 64 remains fixed. FIG. 3A depicts device 60 with elongated shaft 64 in a maximum exposed length (Lₘₐₓ) configuration at which the overall length of device 60 is at a maximum. FIG. 3B depicts device 60 with elongated shaft 64 in a minimum exposed length (Lₘᵢₙ) configuration at which the overall length of device 60 is at a minimum. In certain examples, the ratio of Lₘᵢₙ/Lₘₐₓ ranges from about 0.05 to about 0.20, preferably from about 0.10 to about 0.16, and more preferably from about 0.12 to about 0.14. In one example, the maximum exposed elongated shaft 64 length (Lₘₐₓ) is about 26 cm, the minimum exposed elongated shaft 64 length (Lₘᵢₙ) is about 3.5 cm, and the ratio of Lₘᵢₙ to Lₘₐₓ is about 0.13. In another example, the ratio between the overall device length when the exposed shaft is Lₘᵢₙ to the overall device length when the exposed shaft length is Lₘₐₓ ranges from about 0.25 to about 0.40, preferably from about 0.3 to about 0.36, and more preferably from about 0.32 to about 0.34. In one example, the maximum device length is about 33.7 cm, the minimum device length is about 11 cm, and the ratio of the minimum device length to the maximum device length is about 0.33.

Elongated shaft 64 includes a proximal end 67 (FIG. 4) and a distal end defined by an electrode 38. Handle 62 has an interior lumen 73 within which the proximal elongated shaft end 67 is selectively movable in the device length direction (L) by the user. Electrode 38 is made of a conductive material and is in electrical communication with a source of stimulation energy (not shown). Conductive leads 36 provide stimulation energy to device 60 and are in electrical communication with electrode 38. Because the exposed length of shaft 64 is adjustable, the electrode 38 is selectively repositionable at a plurality of locations with respect to handle 62. In certain examples, the electrode 38 can be securely repositioned at the plurality of locations with respect to handle 62 in the length direction L of device 60.

Electrode 38 may be a mono-polar or bi-polar electrode. In addition, it may have a variety of shapes, including a cylindrical shape with a flat or curved distal end or a wholly or partially spherical shape. In certain examples, and as shown in FIGS. 3A, 3B and 4, the electrode is preferably formed from a medical grade conductive material. In one such example, a known "ball-tip" electrode made of stainless steel is used. In certain examples, SAE (Society of Automotive Engineers) Grade 303 stainless steel is used to form electrode 38.

Elongated shaft 64 is resiliently bendable and provides a conductive path to supply electricity to electrode 38 from a source of stimulation energy (not shown). The elongated shaft 64 may be wholly conductive, partially conductive, or non-conductive. In the case of a non-conductive elongated shaft 64, conductive leads may be provided in a lumen within the shaft 64 to provide a conductive pathway from the source of stimulation energy to electrode 38. In one example, shaft 64 is formed from a medical grade, conductive material and is preferably formed from stainless steel. In one example, SAE Grade 303 stainless steel is used.

Handle 62 is preferably substantially rigid and able to withstand typical gripping forces of an adult human hand. In the example of FIGS. 3A, 3B, and 4, handle 62 has a lumen 73 through which conductive leads 36 such as one or more wires are disposed. Conductive leads 36 are in electrical communication with electrode 38 and a source of stimulation energy (not shown) used to supply electricity to electrode 38. In the illustrated example, elongated shaft 64 is conductive along its length. Thus, conductive leads 36 terminate at and make electrical contact with proximal shaft end 67. To adjust stimulation probe device 60 from the maximum length configuration of FIG. 3A to a shorter configuration, elongated shaft 64 is retracted into the lumen 73 of handle 62. Owing to their flexibility, conductive leads 36 form a collapsible structure that can be compressed within the lumen 73 of handle 62 without compromising their conductivity or disrupting the electrical communication between a source of stimulation energy and electrode 38.

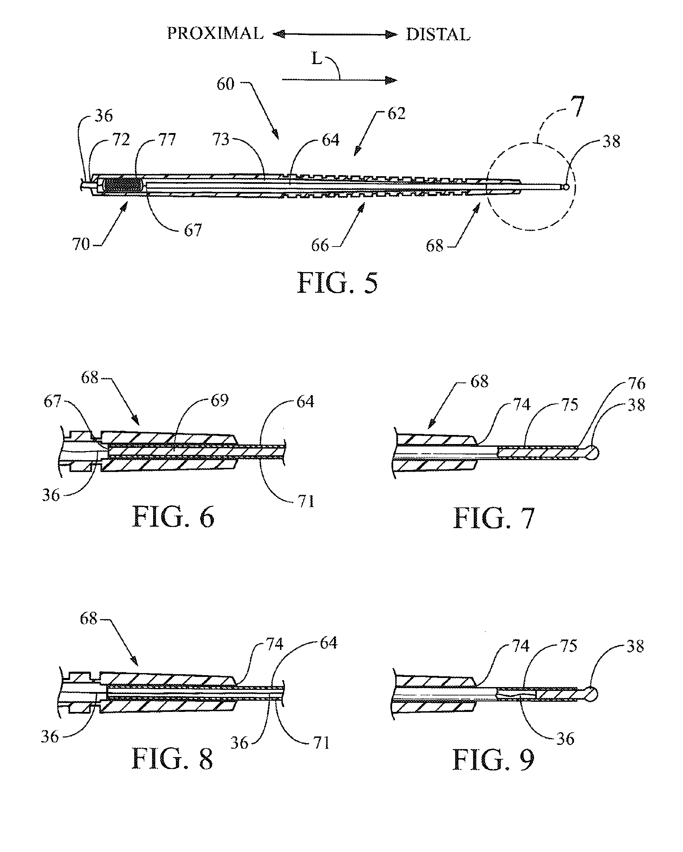

As best seen in FIG. 6, elongated shaft 64 may comprise a radially inner conductive section 69 surrounded by a non-conductive outer annular section 71. This structure beneficially limits the exposed conductive portion of shaft 64 to the electrode 38, which allows the surgeon to isolate and localize the stimulated area of a patient's body to that which is in contact with electrode 38. In certain examples, radially inner conductive section 69 is formed from a medical grade steel such as a medical grade SAE 303 stainless steel. In other examples, non-conductive outer annular section 71 is formed from a medical grade plastic. The non-conductive outer annular section 71 may be coated on radially inner conductive section 69. It may also be separately formed and attached by an adhesive or other means such as heat shrinking. Suitable medical grade plastics include fluorinated polymers such as heat shrinkable PTFE (poly tetrafluoro ethylene, including Teflon^{®}) or FEP (fluorinated ethylene-propylene). In one preferred example, FEP heat shrink tubing is used to form non-conductive outer annular section 71. As shown in FIG. 7, the distal end 38 of elongated shaft 64 may terminate in an electrode which may be integrally formed with shaft 64 or formed separately and then attached thereto.

As best seen in FIG. 8, elongated shaft 64 may alternatively comprise a hollow interior or lumen 79 defined by annular non-conductive section 71. Conductive leads 36 project into the lumen 79 and terminate at electrode 38, as shown in FIG. 9.

In certain examples, elongated shaft 64 has a total length (as measured from proximal shaft end 67 to distal end electrode 38) to outer diameter ratio that ranges from 0.004 to 0.010, preferably from 0.005 to 0.007, and more preferably from 0.0055 to 0.0065. In other examples, elongated shaft 64 has a total length of from about 8 inches (20.3 cm) to about 14 inches (35.6 cm), preferably from about 10 inches (25.4 cm) to about 13 inches (33 cm) and more preferably from about 11.5 inches (29.2 cm) to about 12.5 inches (31.8 cm). In one example, the length is 11.8 inches (30 cm).

In certain examples, elongated shaft 64 has an outer diameter of from about 0.06 (0.15 cm) to about 0.09 inches (0.23 cm), preferably from about 0.068 inches (0.172 cm) to about 0.078 inches (0.20 cm), and more preferably from about 0.070 inches (0.18 cm) to about 0.075 inches (0.10 cm). In one example, the outer diameter is 0.072 inches (0.18 cm).

In certain examples elongated shaft 64 may be selectively extended from and retracted into the lumen 73 of handle 62 to adjust the exposed length of shaft 64 and the overall length of stimulation probe device 60. In some cases, it is preferable to provide a locking device that allows a user to selectively lock the shaft 64 into position once the electrode 38 is spaced apart from handle 62 by a desired distance. In some implementations, distal handle section 68 is configured to provide this locking function. For example, distal handle section 68 may include internal threads that cooperatively engage corresponding threads formed on the outer surface of elongated shaft 64. In this case, the rotation of elongated shaft 64 about its longitudinal axis relative to handle 62 translates elongated shaft 64 along the length direction L with respect to handle 62. It may also be desirable to include surface features such as visual markings on shaft 64 to allow a user to determine the exposed length of shaft 64 and/or the distance by which electrode 38 is spaced apart from the distal handle end 74.

In other examples, stimulation probe device 60 is configured to include an automatically deployable elongated shaft 64 that can be selectively extended from handle 62 by the use of a user interface control device such as button, slider, etc. FIG. 5 depicts one such exemplary structure in which stimulation probe device 60 is provided with an automatic deployment mechanism 77, such as a spring. In FIG. 5, the automatic deployment mechanism 77 preferably provides a conductive path to place conductive leads 36 in electrical communication with elongated shaft 64. A conductive spring, such as one made from medical grade steel is one example of a suitable automatic deployment mechanism 77 for deploying shaft 64.

As best seen in FIG. 6, in the device 60 of FIG. 5, elongated shaft 64 preferably comprises a radially inner conductive section 69 of the type described previously and an annular outer non-conductive section 71 of the type described previously. Conductive leads 36 project away from proximal end 72 of handle 62 and are in electrical contact with automatic deployment mechanism 77. In the embodiment of FIG. 7, automatic deployment mechanism 77 is in a compressed state in which stimulation probe device 60 is at a minimum length corresponding to the minimum exposed shaft 64 length (i.e., elongated shaft 64 is retracted within the lumen 73 of handle 62 to the maximum extent possible). When automatic deployment mechanism 77 is relaxed by releasing it from the compressed state, the stored compressive energy deploys the elongated shaft 64 in the device lengthwise (L) direction, moving shaft 64 into an extended configuration relative to handle 62. The device 60 of FIG. 5 can be configured so that the elongated shaft has two positions, respectively corresponding to a compressed and a relaxed automatic deployment mechanism state. However, in other embodiments, the automatic deployment mechanism 77 may have a number of intermediate states between fully compressed and fully relaxed, each of which corresponds to a different spacing between electrode 38 and handle distal end 74. Suitable buttons, slider switches, and other user controls known to those skilled in the art may be used to deploy elongated shaft 64 to one or more positions relative to handle 62.

Other automatic deployment mechanisms 77 may also be used to automatically deploy elongated shaft 64 relative to handle 62. For example, a motor that is selectively energized by a user switch on device 60 or elsewhere may be operatively connected to shaft 64 to extend and retract it relative to handle 62. In certain configurations, it may be desirable to configure device 60 so that the elongated shaft 64 is movable to a plurality of discrete, spaced apart indexed positions, including positions that are spaced apart by a uniform distance. This design assists the user in varying the exposed length of shaft 64 relative to handle 62 by fixed incremental amounts to provide a more predictable extension and retraction of the shaft 64 relative to handle 62. In certain implementations, a transition to an indexed position may be accompanied by an indexed movement sound such as an audible click.

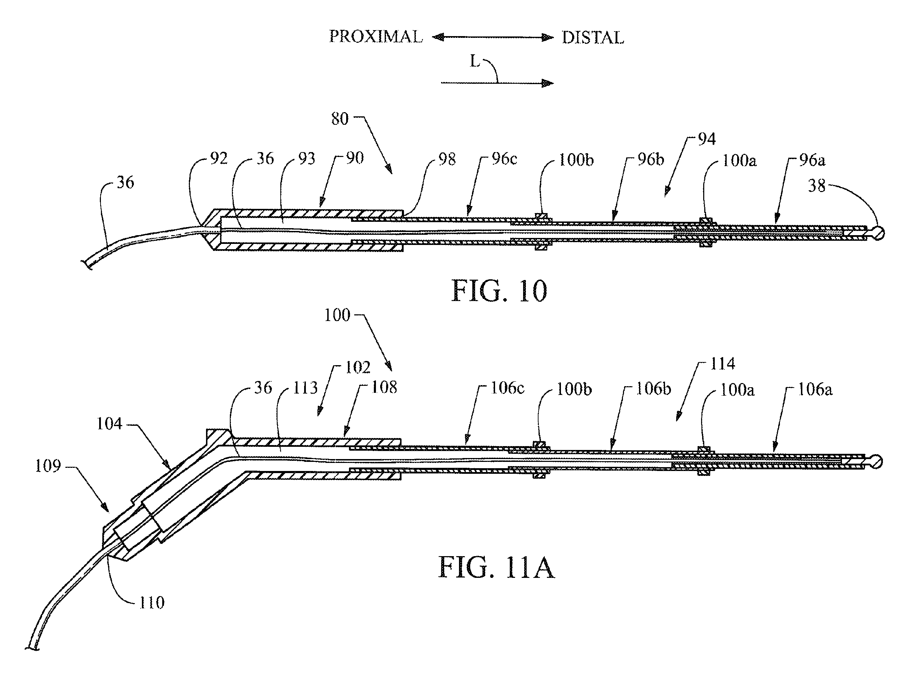

Another version of a stimulation probe device with an adjustable length is depicted in FIG. 10. Stimulation probe device 80 includes a handle 90 connected to an elongated shaft that comprises an adjustable sheath 94. Electrode 38 is provided at the distal most end of sheath 94. Sheath 94 includes a plurality of hollow tube segments 96a, 96b, and 96c. In the embodiment of FIG. 10, sheath 94 is a telescoping sheath comprised of insulating material, in which proximal sheath portion 96c retracts into handle 90, while middle sheath portion 96b retracts into proximal sheath portion 96c, and distal sheath portion 96a retracts into middle sheath portion 96b. The individual sheath portions 96c to 96a may be individually deployed to adjust the length of sheath 94 to a desired length. In one example, proximal sheath portion 96c may be fully retracted into handle 90, middle sheath portion 96b may be fully retracted into proximal sheath portion 96c, and distal sheath portion 96a may be partially or fully retracted into middle sheath portion 96b. In another example, proximal sheath portion 96c may be fully retracted into handle 90, middle sheath portion 96b may be fully extended from proximal sheath portion 96c, and distal sheath portion 96a may be fully extended from middle sheath portion 96c. In yet another example, all three sheath portions 96a-c may be fully retracted. In addition, individual sheath portions may be partially retracted.

Electrode 38 is in electrical communication with a source of stimulation energy (not shown). Conductive leads 36 enter handle 90 at proximal end 92 and run through the lumen 93 of handle 90 and sheath lumen 103, terminating at electrode 38. Electrode 38 may be mono-polar or bi-polar. It may also have a cylindrical shape with a generally flat distal face or may be partially or wholly spherical. The example of FIG. 10, the electrode 38 is a ball-tip electrode of the type known in the art.

In order to ensure that the length of stimulation probe device 80 stays at the user-selected length, one or more releasable sheath connectors 100a, 100b may be used. In one example, the releasable sheath connectors 100a and 100b are twist lock devices. Such twist lock devices can be rotated to allow the sheath sections 96a-96c to be extended or retracted as desired. They can then be rotated to secure the sheath sections into place.

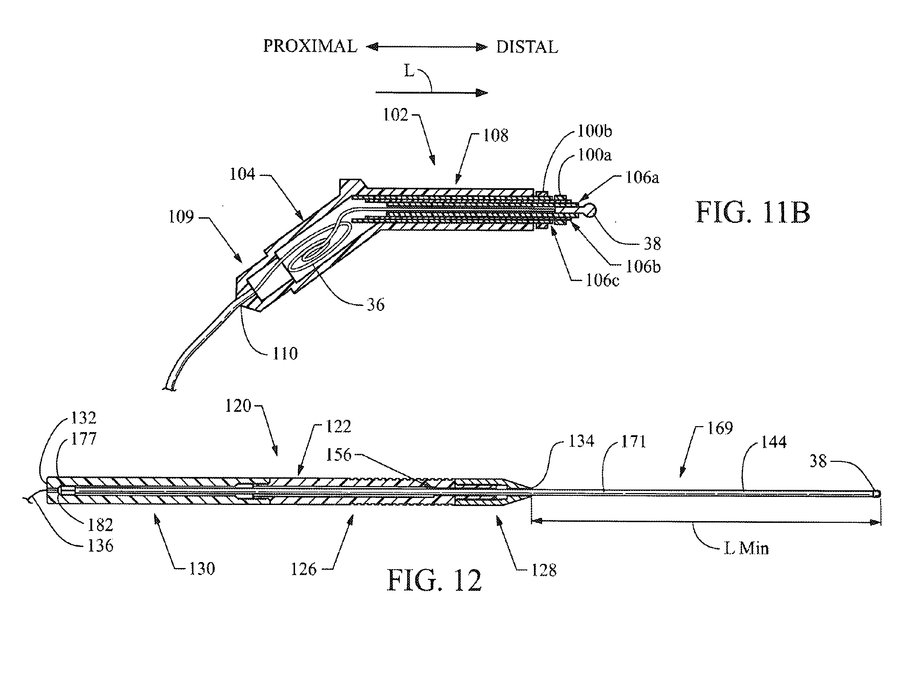

Referring to FIGS. 11A and 11B, another embodiment of a stimulation probe device with an adjustable length is shown. Stimulation probe device 100 includes a handle 102 and an elongated shaft comprising an insulated adjustable sheath 114 similar to the adjustable sheath 94 of FIG. 10. Adjustable sheath 114 is telescoping and comprises a proximal section 106c, a middle section 106b, and a distal section 106a. Proximal sheath section 106c is selectively retractable into and extendable from the interior of handle 102. Middle sheath section 106b is selectively retractable into and extendable from the interior of proximal sheath section 106c. Distal sheath section 106a is selectively retractable into and selectively extendible from the interior of middle sheath section 106b. Releasable sheath connectors 100a and 100b connect middle sheath section 106b to distal sheath section 106a and proximal sheath section 106c, respectively.

FIG. 11A shows stimulation probe device 100 at its maximum length, with each sheath section 106a-106c in a fully extended position. FIG. 11B shows stimulation probe device 100 at its minimum length, with each sheath section 106a-106c in a fully retracted position. Although FIG. 10 is not shown in a retracted configuration, in that configuration sheath 94 would appear substantially similarly to sheath 114 of FIG. 11B.

The handle 102 of stimulation probe device 100 is angled. Distal handle portion 108 is substantially co-axially aligned with sheath 114. However, middle handle portion 104 is angled with respect to distal handle portion 108. The angle defined between handle portions 104 and 108 is generally obtuse. Preferred angles range from about 110 degrees to about 160 degrees. Angles from about 125 degrees to about 145 degrees are more preferred, and angles from about 130 degrees to about 140 degrees are especially preferred. In the example of FIGS. 11A and 11B, the angle between distal handle section 108 and middle handle section 104 is about 135 degrees. Handle 102 also includes a proximal section 109 that projects away from middle handle section 104 and which includes a port 110 in which conductive leads 36 are received.

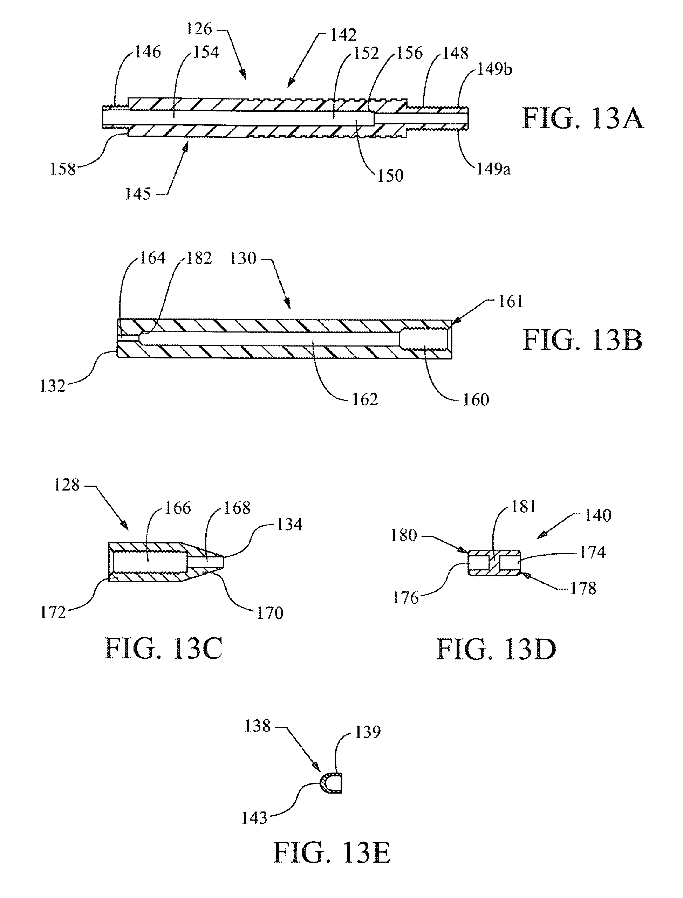

Referring to FIGS. 12 and 13A-13E, another stimulation probe device 120 is shown and described. Stimulation probe device 120 includes a substantially straight handle 122 and a substantially straight elongated shaft 144. Elongated shaft 144 is movably disposed within a lumen or opening extending along the length of handle 122.

Handle 122 includes a proximal end 132 and a distal end 134. Handle 122 also comprises proximal section 130, a middle section 126 and distal section 128. In the figures, each section is separately formed and then subsequently attached. However, the three sections may also be integrally formed (e.g., molded) as a single piece. Handle 126 is preferably substantially rigid and made of a plastic, and more preferably, a medical grade thermoplastic. In one example, a polyoxymethylene such as an acetal plastic homopolymer or copolymer is used.

Referring to FIG. 13B, proximal lumen 162 of proximal handle section 130 opens into a distal lumen 160. Proximal handle section 130 also includes a conductive lead lumen 164 through which conductive leads 36 (not shown) are disposed. Conductive lead lumen 164 is in communication with proximal lumen 162 at tapered section 182. Thus, conductive lead lumen 164 generally has a smaller diameter than proximal lumen 162.

Referring to FIG. 13A, middle handle section 126 includes a proximal neck 146, a first intermediate portion 145, a second intermediate portion 142, and a distal neck 148. Distal lumen 160 in proximal handle section 130 is sized to receive and engage proximal neck 146 of middle handle section 126 to connect proximal handle section 130 to middle handle section 126. Proximal neck 146 may snap fit into distal lumen 160 or may be attached by threaded engagement, an adhesive, or mechanical fasteners. Shoulder 158 may abuttingly engage distal face 161 of proximal handle section 130 to restrain the relative movement of proximal handle section 130 and middle handle section 126.

Distal handle section 128 includes a proximal lumen 166 within proximal portion 172 of distal handle section 128 which receives distal neck 148 of middle handle section 126. In the illustrated embodiment, distal neck 148 of middle handle section 126 is tapered down to a lower outer diameter than that of middle portion 142 of middle handle section 126. In one example, distal neck 148 includes outer surface threads that engage complementary threads formed on the inner surface of proximal portion 172 of distal handle section 128. This configuration allows distal handle section 128 to threadingly engage and disengage distal neck 148 of middle handle section 126. Proximal lumen 166 of distal handle section 128 is in communication with a distal lumen 168 in distal portion 170 of distal handle section 128 through which elongated shaft 144 projects.

Stimulation probe device 120 has an adjustable length that is adjusted by extending or retracting elongated shaft 144 relative to handle 122, thereby adjusting the exposed length of shaft 144. In the depicted embodiment, elongated shaft 144 can be extended along a continuum of positions relative to handle 122 in a smooth sliding motion. In a preferred embodiment, and as illustrated in FIGS. 12 and 13A-13E, a locking device is provided in distal handle section 128 to releasably lock the position of elongated shaft 144 and its electrode 138 relative to handle 122. In the illustrated embodiment, distal handle section 128 is formed as an adjustable chuck and distal neck 148 of middle handle section 126 is resiliently compressible and expandable so that as the distal handle section 128 threadingly engages distal neck 148 of middle handle section 126, the diameter of distal neck 148 is constricted to frictionally engage elongated shaft 144, thereby holding the shaft 144 in place relative to handle 122.

In one example, distal neck 148 of middle handle section 126 is configured with a plurality of longitudinal slots which allow the distal neck 148 to compress against elongated shaft 144 as distal handle section 128 engages distal neck 148 of middle handle section 126. In accordance with the example, distal handle section 128 is rotatably loosened from distal neck 148 of middle handle section 126 to allow distal neck 148 to relax and expand radially. A user then slides elongated shaft 144 to position electrode 138 at the desired location relative to handle 122. Distal handle section 128 is then rotatably tightened to distal neck 148 of middle handle section 126, thereby radially compressing distal neck 148 and securing the elongated shaft 144 at the desired location relative to distal end 134 of handle 122.

Elongated shaft 144 may be similar to the shaft 64 described previously. However, in the embodiment of FIGS. 12 and 13A-13E, shaft 144 preferably comprises an inner conductive section 169 and an outer, annular non-conductive section 171. In one example, elongated shaft 144 comprises an inner conductive section 169 formed from medical grade stainless steel, such as a medical grade SAE 303 stainless steel, and an outer annular section 171 formed from a non-conductive plastic such as a heat shrinkable fluoropolymer of the type described previously.

Handle 122 and elongated shaft 144 are preferably configured to securely retain elongated shaft 144 to handle 122 while allowing the elongated shaft 144 to retract into and extend from handle 122. In certain implementations, elongated shaft 144 has a first shaft stop surface 178 (FIGS 12 and 13D) between its proximal end 177 and its distal end 138, and handle 122 has a first handle stop surface 156 (FIG. 13A) between its proximal end 132 and its distal end 134. First handle stop surface 156 separates proximal lumen 154 from distal lumen 150. In other implementations, elongated shaft 144 has a second stop surface 180 (FIGS. 12 and 13D) on its proximal end 177 or between its proximal end 177 and distal end 138, and the handle 122 has a second stop surface 182 (FIGS. 12 and 13B) at its proximal end 132 or between its proximal end 132 and distal end 134. The engagement of the first shaft and handle stop surfaces 178 and 156 retains elongated shaft 144 to handle 122 and prevents a user from pulling elongated shaft 144 entirely out of distal end 134 of handle 122. The engagement of second shaft and handle stop surfaces 180 and 182 limits the retraction of elongated shaft 144 within handle 122 and prevents a user from pulling elongated shaft 144 entirely out of proximal end 132 of handle 122.

In one example, first and second shaft stop surfaces 178 and 180 are defined on a conductive stop 140 (FIG. 13D) adjacent to or at the proximal end 177 of elongated shaft 144. Conductive stop 140 includes a proximal end opening 176 into which conductive leads 36 enter to connect to contact surface 181. Contact surface 181 separates proximal end opening 176 from a distal end opening 174 that receives and engages a proximal portion of the elongated shaft 144. Thus, electrical leads 36 are in electrical communication with the inner conductive section 169 of elongated shaft 144 via electrical contact made at contact surface 181. Distal opening 174 of conductive stop 140 is defined by an annular distal end face that defines first shaft stop 178.

Proximal opening 176 of conductive stop 140 is defined by an annular proximal end face that defines second shaft stop surface 180. In the illustrated example, second handle stop surface 182 (FIGS. 12 and 13B) is a tapered portion of the inner surface of proximal handle portion 130 that narrows proximal lumen 162. First handle stop surface 156 is defined by an annular proximally facing contact surface formed in the interior of middle handle section 126. As indicated previously, in the example shown in FIGS. 12 and 13A-13E, elongated shaft 144 is retained by handle 122 and is not separable therefrom. However, other implementations may be used in which shaft 144 is separable. In additional implementations, a variety of elongated shafts 144 of different lengths may be selectively attached to handle 122 to provide an adjustable length stimulator probe kit with a stimulator probe handle and a variety of shafts 144, each of which his separately attachable to the handle to provide their own corresponding minimum and maximum exposed shaft 144 lengths.

In FIG. 12, stimulation probe device 120 is depicted in a minimum length configuration with the minimum exposed length (Lₘᵢₙ) extending away from distal end 134 of handle 122. As shown in FIG. 12, second shaft stop surface 180 is in abutting engagement with second handle stop surface 182 of proximal lumen 162 in proximal handle section 130, which restrains any further retraction of elongated shaft 144 within handle 122. To extend elongated shaft to its maximum exposed length, a user would loosen the distal handle section 128 (which is an adjustable chuck in the example of FIGS. 12 and 13C) from the distal neck 148 of middle handle section 126 and slide elongated shaft 144 in the distal direction until first shaft contact surface 178 of conductive stop 140 engages first handle contact surface 156 within middle handle section 126, thereby restraining any further distal movement of elongated shaft 144 relative to handle 122.

Electrode 138 may be monopolar or bipolar. It may also be cylindrical, annular, or spherical. As depicted in FIG. 13E, in one example, the electrode 138 is a ball-tip electrode with a spherical portion 143 having a radius of curvature ranging from about 0.05 cm (0.020 inches) to about 0.2 cm (0.08 inches), preferably from about 0.08 cm (0.03 inches) to about 0.13 cm (0.05 inches), and more preferably from about 0.107 cm (0.042 inches) to about 0.122 cm (0.048 inches). In one example, the radius of curvature is about 0.117 cm (0.046 inches). Electrode 138 includes an annular portion 139 that is connectable to a portion of elongated shaft 144. Electrode 138 is preferably formed from a medical grade stainless steel such as SAE 303 stainless steel. Thus, the connection between electrode 138, the inner conductive section 169 of elongated shaft 144, and conductive end stop 140 provides a conductive path through stimulation probe 120 that allows electrode 138 to be placed in electrical communication with a source of stimulation energy.

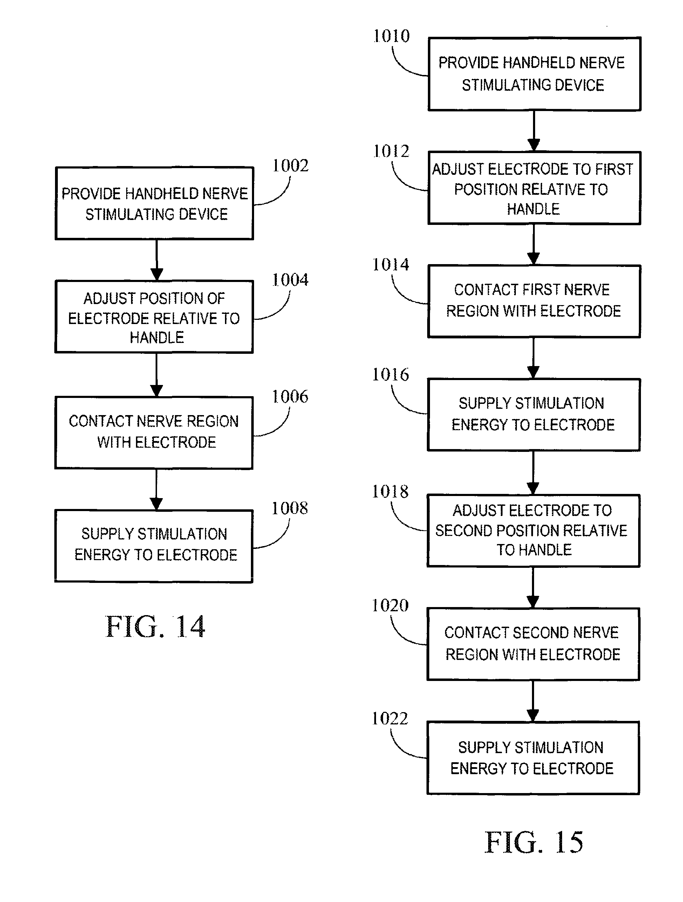

Referring to FIG. 14 a method of using an adjustable length stimulation probe of the type described previously will now be described. In accordance with the method, the stimulation probe device is used to supply stimulation energy to a nerve region in which nerves are suspected or confirmed to be located. The stimulation may be used for a variety of purposes, including to perform proactive nerve location and/or to perform nerve integrity impairment monitoring. In accordance with the method, in step 1002 a handheld nerve stimulating device is provided, such as devices 60, 80, 100, or 120. If a stimulation probe device kit of the type described previously is used, one of its elongated shafts is attached to the handle 62, 90, 102, 122 in step 1002.

In step 1004, the position of the device electrode (e.g., electrode 38 or 138) is adjusted relative to the handle 62, 90, 102, 122 of the device 60, 80, 100, 120 to adjust the overall device length as desired. In step 1006, the user contacts a nerve region in or on patient's body with the electrode 38, 138. For example, a surgeon may make the necessary incisions in the patient to access the tissue of interest. The surgeon then grips the handle (62, 90, 102, 122) of the device (60, 80, 100, 120) with a single hand to hold the device (60, 80, 100, 120) and moves the electrode (38, 138) to a nerve region where nerves are suspected to be located (for proactive nerve location) or have been confirmed to have been located (for nerve integrity impairment monitoring). The surgeon then contacts the electrode 38, 138 with the tissue to make physical and electrical contact.

In step 1008, stimulation energy is supplied to the electrode (38, 138). To perform step 1008, a separate stimulation energy device (not shown) may be placed in electrical communication with the electrode 38, 138 such as by connecting conductive leads 36 emanating from the stimulation probe device 60, 80, 100, 120 to the stimulating energy device.

In certain examples, a recording sensor will be placed in the nerve region to sense any evoked responses created by supplying stimulation energy. After the recording sensor detects evoked nerve responses, it will subsequently transmit the nerve response feedback to an IONM system generator, which in turn, will alert the surgeon and/or IONM clinician to an at risk nerve via audio tones and EMG waveform visualization technologies.

In certain implementations, the recording sensor will use preprogrammed, evoked nerve response electrical stimulation intensity and frequency delivery level recordings, as a standard for comparison of the evoked response. The preprogrammed, evoked nerve response recordings will comprise significantly lower electrical stimulation intensity and frequency delivery levels typically used by surgeons to evoke CAPs e.g. an electrical stimulation delivery range between 0.5mA - 5.0mA. In the surgical environment, the human body will only produce a CAP when a motor nerve is electrically stimulated by a surgeon while performing IONM. When a CAP is electronically evoked by the surgeon there is a dramatic, quantifiable, "spike" in the electrical stimulation and frequency intensity levels, particularly in the surgical field, where the CAP is manually evoked by the surgeon. For example, it is not uncommon for peak CAP stimulation intensity levels to be greater than 150mA. As previously stated, the recording sensor will be preprogrammed to specifically listen for characteristic CAP events, in terms of their electrical stimulation and frequency intensity spike levels, in comparison to the relatively insignificant 0.5mA - 5mA of electrical stimulation intensity delivered by the surgeon to the surgical field when performing IONM.

Prior to or immediately following the supplying of stimulation energy in step 1008, an EMG monitoring unit (not shown) may be activated to receive and store (in data storage such as a hard drive, flash drive, etc.) any evoked signals generated by nerves that are stimulated by the supplied stimulation energy. A processor then executes programmed instructions to evaluate evoked signals and determine whether a compound action potential has been generated. In nerve integrity impairment monitoring applications, the programmed instructions are configured to determine whether the compound action potential is indicative of nerve integrity impairment. In one example, the programmed instructions compare the evoked signals received from the patient to baseline evoked nerve response data stored in data storage to determine whether nerve integrity has been impaired. For example, evoked nerve response signals may be collected from a patient prior to surgery to determine a baseline evoked nerve response when the nerve integrity is known to be unimpaired. Differences from the baseline condition may indicate that nerve integrity has been compromised. In one embodiment, differences between evoked nerve signals received from an EMG nerve sensor and the baseline data are calculated by subtraction to determine the level of variation from the baseline response. This technique also beneficially mitigates the effects that the stimulation energy may have on the EMG nerve sensor independent of any evoked nerve response. Signal filtering techniques known to those skilled in the art may also be used to remove noise from the signals provided by the recording sensor.

The processor may be programmed to compare the evoked nerve response data provided by an EMG nerve response sensor to baseline data. However, when locating nerves, it is the generation of a compound action potential that is most significant, whereas in nerve integrity monitoring, changes in compound action potentials are generally more significant. Thus, in certain nerve locating applications "baseline" signal to which sensed evoked nerve signals are compared may simply be the stimulation signal supplied to nerve stimulating electrode 38, 138. The use of such a baseline signal filters out the effects of stimulation energy that is received directly by the EMG nerve sensor, as opposed to stimulation potentials that are evoked in response to nerve stimulation.

In certain methods, it may be desirable to use stimulation probe devices having multiple lengths during a single surgical procedure. The adjustable length stimulation probe devices 60, 80, 100, 120 are particularly useful in such procedures because they allow a single device to be used, thereby reducing the complexity of the procedure and--at least in the case of disposable devices--reducing waste. Referring to FIG. 15, a method of using an adjustable length stimulation probe device is illustrated in which the device is adjusted to multiple lengths during a procedure. In accordance with the method, a handheld, adjustable length stimulation probe device 60, 80, 100, 120 is provided. In step 1012, the electrode 38, 138 is adjusted to a first position relative to the device handle 62, 90, 102, 122 by adjusting the position of the elongated shaft 64, 94,114, 144 relative to the handle 62, 90, 102, 122. If a locking device is provided, it is then used to fix the position of the elongated shaft 65, 94, 114, 144 and electrode 38, 138 relative to the handle 62, 90, 102, 122.

In step 1014, the electrode 38, 138 is placed in contact with a first nerve region in or on the patient's body. In step 1016, stimulation energy is supplied to the electrode 38, 138, thereby stimulating the nerve region. At this point, an EMG sensor may be used to sense an evoked nerve response and determine if nerves are located in the nerve region (in the case of proactive nerve location procedures) or if the integrity of nerves has been impaired (in the case of nerve integrity impairment monitoring).

At this point, the surgeon may perform a surgical procedure (e.g., removing or repairing tissue, organs, etc.). In step 1018, the position of electrode 38, 138 relative to handle 62, 90, 102, 122 is adjusted by adjusting the position of elongated shaft 64, 94, 114, 144 relative to stimulation probe device handle 62 90, 102, 122 in the manner described previously. In certain cases, stimulation probe device 60, 80, 100, 120 may be configured for indexed extension and retraction of elongated shaft 64, 94, 114, 144 relative to handle 62, 90, 102, 122, in which case step 1018 may comprise adjusting the position of elongated shaft 64, 94, 114, 144 relative to handle 62, 90, 102, 122 by an indexed amount. If device 60, 80, 100, 120 includes an audible indexed movement indication (e.g., an audible click), the surgeon may use the number of such indications (e.g., a number of clicks) to determine the extent to which the elongated shaft 144 has been extended or retracted relative to handle 122.

In step 1020, a second nerve region is contacted with the electrode 38, 138 (step 1020) and stimulation energy is supplied (step 1022). An EMG recording sensor may again be used to determine if an evoked response indicates that a nerve is present (proactive nerve location) or impaired (nerve integrity impairment monitoring). The first and second nerve regions are preferably spaced apart from one another in and/or on the patient's body. In certain examples, the first nerve region is spaced apart from a surface of the patient's body by a first distance, the second nerve region is spaced apart from the surface of the patient's body by a second distance, and the second distance is greater than the first distance.

In one example, one or both nerve regions are proximate a portion of the lumbar spine, as would be the case during an XLIF (extreme lateral interbody fusion) procedure. In such cases, the first nerve region may be proximate one vertebra and the second may be proximate another vertebra, and the surgeon may use the stimulation probe device to first locate nerves proximate each vertebra and then remove an intervertebral disc once the nerves have been located. An interbody spacer may then be placed between the vertebrae.

The present invention has been described with reference to certain exemplary embodiments thereof. However, it will be readily apparent to those skilled in the art that it is possible to embody the invention in specific forms other than those of the exemplary embodiments described above.

The exemplary embodiments are merely illustrative and should not be considered restrictive in any way. The scope of the invention is defined by the appended claims and their equivalents, rather than by the preceding description.

## Claims

1. A handheld nerve stimulating device (60, 80) with an adjustable length, comprising:
a handle (62, 90); and
an elongated shaft (64, 94) having a distal end with an electrode (38) made of a conductive material and in electrical communication with a source of stimulation energy,
wherein the elongated shaft is selectively movable with respect to the handle; wherein the overall length of the device is adjustable by adjusting the exposed length of the elongated shaft projecting away from a distal end (74, 98) of the handle while the length of the handle remains fixed;
wherein:
the elongated shaft (64) comprises a radially inner conductive section (69) surrounded by a non-conductive outer annular section (71), so that the exposed conductive portion of the elongated shaft (64) is limited to the electrode (38), or
the elongated shaft (64, 94) comprises a hollow interior or lumen defined by an annular non-conductive section (71); **characterized in that**
the handle includes a proximal end and a distal end, the distal end of the handle is between the electrode and the proximal end of the handle, the device further comprises a locking device that engages a portion of the handle, and the locking device includes a tightened configuration and a loosened configuration, such that when the locking device is in the tightened configuration, the elongated shaft is immovable with respect to the handle, and when the locking device is in the loosened configuration, the shaft is movable with respect to the handle.

2. The device of claim 1, wherein the handle has an interior (73), the elongated shaft has a proximal end (67), and the proximal end is movable within the interior of the handle.

3. The device of claim 1, wherein the electrode is selectively and securely repositionable at a plurality of locations with respect to the handle.

4. The device of claim 1, wherein the handle has a lumen interior, a proximal end, and a spring in the lumen at the proximal end of the handle, the elongated shaft has a retracted position relative to the handle and an extended position relative to the handle, the spring has a compressed configuration and a relaxed configuration, and when the spring is in a relaxed configuration, the shaft is in the extended position relative to the handle.

5. The device of claim 1, further comprising a motor operatively connected to the shaft, wherein the motor is selectively energizable to move the elongated shaft relative to the handle.

6. The device of claim 1, wherein the electrode is a ball-tip electrode.

7. The device of claim 1, further comprising a user control operatively connected to the elongated shaft, such that movement of the user control causes the elongated shaft to move relative to the handle.

8. The device of claim 1, wherein the elongated shaft is selectively movable to a plurality of indexed positions, and each indexed position corresponds to a fixed distance between the electrode and the handle.

9. The device of claim 8, wherein as the elongated shaft moves from one indexed position to another indexed position, an indexed movement sound is emitted.

10. The device of claim 1, wherein the elongated shaft includes a plurality of surface features indicative of the distance between the electrode and the handle.

11. The device of claim 1, wherein the electrode has a maximum extended position defining a first distance between the electrode and the handle and a minimum extended position defining a second distance relative to the handle, and the ratio between the second distance and the first distance is from about 0.05 to about 0.2.

12. The device of claim 1, wherein the handle has a proximal end and a distal end, the distal end of the handle is located between the electrode and the proximal end of the handle, and the electrode is in electrical communication with a lead wire extending from the proximal end of the handle.

13. The device of claim 1, wherein the elongated shaft has a proximal end and a distal end and includes a shaft stop surface between the shaft proximal end and the shaft distal end, the handle has a proximal end and a distal end and includes a handle stop surface between the handle proximal end and the handle distal end, such that the engagement of the shaft stop surface and the handle stop surface retains the shaft to the handle.

14. The device of claim 1, wherein the shaft is selectively detachable from the handle.

## Patentansprüche

1. Eine handgeführte Nervenstimulationsvorrichtung (60, 80) mit einer einstellbaren Länge, aufweisend:
einen Griff (62, 90) und
einen länglichen Schaft (64, 94), welcher ein distales Ende mit einer Elektrode (38) hat, welche aus einem leitfähigen Material hergestellt ist und in elektrischer Verbindung mit einer Stimulationsenergiequelle ist,
wobei der längliche Schaft in Bezug auf den Griff selektiv bewegbar ist, wobei die Gesamtlänge der Vorrichtung einstellbar ist durch Einstellen der freiliegenden Länge des länglichen Schafts, welcher von einem distalen Ende (74, 98) des Griffs weg vorspringt, während die Länge des Griffs fixiert bleibt,
wobei:
der längliche Schaft (64) einen radial inneren leitfähigen Abschnitt (69) aufweist, welcher von einem nicht-leitfähigen äußeren ringförmigen Abschnitt (71) umgeben ist, so dass der freiliegende leitfähige Abschnitt des länglichen Schafts (64) auf die Elektrode (38) limitiert ist, oder
der längliche Schaft (64, 94) ein hohles Inneres oder Lumen aufweist, welches durch einen ringförmigen nicht-leitfähigen Abschnitt (71) definiert ist, **dadurch gekennzeichnet, dass**
der Griff ein proximales Ende und ein distales Ende aufweist, das distale Ende des Griffs zwischen der Elektrode und dem proximalen Ende des Griffs ist, die Vorrichtung ferner eine Feststellvorrichtung aufweist, welche mit einem Abschnitt des Griffs in Eingriff ist, und die Feststellvorrichtung eine festgestellte Konfiguration und eine gelöste Konfiguration aufweist, so dass der längliche Schaft in Bezug auf den Griff nicht bewegbar ist, wenn die Feststellvorrichtung in der festgestellte Konfiguration ist, und der Schaft in Bezug auf den Griff bewegbar ist, wenn die Feststellvorrichtung in der gelösten Konfiguration ist.

2. Die Vorrichtung nach Anspruch 1, wobei der Griff ein Inneres (73) hat, der längliche Schaft ein proximales Ende (67) hat und das proximale Ende innerhalb des Inneren des Griffs bewegbar ist.

3. Die Vorrichtung nach Anspruch 1, wobei die Elektrode in einer Mehrzahl von Positionen in Bezug auf den Griff selektiv und sicher wiederpositionierbar ist.

4. Die Vorrichtung nach Anspruch 1, wobei der Griff ein Lumen-Inneres, ein proximales Ende und eine Feder im Lumen an dem proximalen Ende des Griffs hat, der längliche Schaft eine eingefahrene Position relativ zu dem Griff und eine ausgefahrene Position relativ zu dem Griff hat, die Feder eine zusamrnengedrückte Konfiguration und eine entspannte Konfiguration hat und der Schaft in der ausgefahrenen Position relativ zu dem Griff ist, wenn die Feder in einer entspannten Konfiguration ist.

5. Die Vorrichtung nach Anspruch 1, ferner aufweisend einen Motor, welcher wirksam mit dem Schaft verbunden ist, wobei der Motor selektiv mit Energie versorgbar ist, um den länglichen Schaft relativ zu dem Griff zu bewegen.

6. Die Vorrichtung nach Anspruch 1, wobei die Elektrode eine Kugelspitze-Elektrode ist.

7. Die Vorrichtung nach Anspruch 1, ferner aufweisend eine Verwendersteuerung, welche wirksam mit dem länglichen Schaft verbunden ist, so dass Bewegung der Verwendersteuerung Bewegung des länglichen Schafts relativ zu dem Griff bewirkt.

8. Die Vorrichtung nach Anspruch 1, wobei der längliche Schaft selektiv zu einer Mehrzahl von indizierten Positionen bewegbar ist und jede indizierte Position einer fixierten Distanz zwischen der Elektrode und dem Griff entspricht.

9. Die Vorrichtung nach Anspruch 8, wobei ein indiziertes Bewegungsgeräusch ausgegeben wird, wenn der längliche Schaft von einer indizierten Position zu einer anderen indizierten Position bewegt wird.

10. Die Vorrichtung nach Anspruch 1, wobei der längliche Schaft eine Mehrzahl von Flächenmerkmalen aufweist, welche die Distanz zwischen der Elektrode und dem Griff anzeigen.

11. Die Vorrichtung nach Anspruch 1, wobei die Elektrode eine maximale ausgefahrene Position, welche eine erste Distanz zwischen der Elektrode und dem Griff definiert, und eine minimale ausgefahrene Position hat, welche eine zweite Distanz relativ zu dem Griff definiert, und das Verhältnis zwischen der zweiten Distanz und der ersten Distanz von etwa 0,05 bis etwa 0,2 ist.

12. Die Vorrichtung nach Anspruch 1, wobei der Griff ein proximales Ende und ein distales Ende hat, das distale Ende des Griffs zwischen der Elektrode und dem proximalen Ende des Griffs angeordnet ist und die Elektrode in elektrischer Verbindung mit einer Anschlussleitung ist, welche sich von dem proximalen Ende des Griffs erstreckt.

13. Die Vorrichtung nach Anspruch 1, wobei der längliche Schaft ein proximales Ende und ein distales Ende hat und eine Schaft-Stopp-Fläche zwischen dem proximalen Ende des Schafts und dem distalen Ende des Schafts aufweist, der Griff ein proximales Ende und ein distales Ende hat und eine Griff-Stopp-Fläche zwischen dem proximalen Ende des Griffs und dem distalen Ende des Griffs aufweist, so dass der Eingriff der Schaft-Stopp-Fläche und der Griff-Stopp-Fläche den Schaft an dem Griff festhält.

14. Die Vorrichtung nach Anspruch 1, wobei der Schaft selektiv von dem Griff abnehmbar ist.

## Revendications

1. Dispositif à main de stimulation d'un nerf (60, 80) qui présente une longueur réglable, comprenant :
une poignée (62, 90) ; et
une tige allongée (64, 94) qui présente une extrémité distale avec une électrode (38) réalisée dans un matériau conducteur, et en communication électrique avec une source d'énergie de stimulation ;
dans lequel la tige allongée est mobile de manière sélective par rapport à la poignée ;
dans lequel la longueur totale du dispositif est réglable en réglant la longueur exposée de la tige allongée qui fait saillie à partir d'une extrémité distale (74, 98) de la poignée tandis que la longueur de la poignée demeure fixe ;
dans lequel :
la tige allongée (64) comprend une section conductrice intérieure de manière radiale (69) entourée par une section annulaire extérieure non conductrice (71), de telle sorte que la partie conductrice exposée de la tige allongée (64) soit limitée à l'électrode (38) ; ou
la tige allongée (64, 94) comprend une lumière ou un intérieur creux défini par une section non conductrice annulaire (71) ;
**caractérisé en ce que** la poignée comprend une extrémité proximale et une extrémité distale, l'extrémité distale de la poignée se situe entre l'électrode et l'extrémité proximale de la poignée, le dispositif comprend en outre un dispositif de verrouillage qui vient en prise avec une partie de la poignée, et le dispositif de verrouillage comprend une configuration serrée et une configuration desserrée, de telle sorte que, lorsque le dispositif de verrouillage se trouve dans la configuration serrée, la tige allongée soit immobile par rapport à la poignée, et que, lorsque le dispositif de verrouillage se trouve dans la configuration desserrée, la tige soit mobile par rapport à la poignée.

2. Dispositif selon la revendication 1, dans lequel la poignée présente un intérieur (73), la tige allongée présente une extrémité proximale (67), et l'extrémité proximale est mobile à l'intérieur de l'intérieur de la poignée.

3. Dispositif selon la revendication 1, dans lequel l'électrode peut être repositionnée de manière sélective et fixe au niveau d'une pluralité d'emplacements par rapport à la poignée.

4. Dispositif selon la revendication 1, dans lequel la poignée présente une lumière, une extrémité proximale, et un ressort dans la lumière au niveau de l'extrémité proximale de la poignée, la tige allongée présente une position rentrée par rapport à la poignée et une position sortie par rapport à la poignée, le ressort présente une configuration comprimée et une configuration détendue, et lorsque le ressort se trouve en configuration détendue, la tige se trouve dans la position sortie par rapport à la poignée.

5. Dispositif selon la revendication 1, comprenant en outre un moteur connecté de manière opérationnelle à la tige, dans lequel le moteur peut être alimenté de manière sélective de façon à déplacer la tige allongée par rapport à la poignée.

6. Dispositif selon la revendication 1, dans lequel l'électrode est une électrode dont le bout est une sphère.

7. Dispositif selon la revendication 1, comprenant en outre une commande utilisateur connectée de manière opérationnelle à la tige allongée, de telle sorte qu'un mouvement de la commande utilisateur provoque un déplacement relatif de la tige allongée vers la poignée.

8. Dispositif selon la revendication 1, dans lequel la tige allongée est mobile de manière sélective vers une pluralité de positions indexées, et chaque position indexée correspond à une distance fixe entre l'électrode et la poignée.

9. Dispositif selon la revendication 8, dans lequel, lorsque la tige allongée se déplace à partir d'une position indexée vers une autre position indexée, un son de déplacement indexé est émis.

10. Dispositif selon la revendication 1, dans lequel la tige allongée comprend une pluralité de caractéristiques de surface indicatives de la distance entre l'électrode et la poignée.

11. Dispositif selon la revendication 1, dans lequel l'électrode présente une position sortie maximum qui définit une première distance entre l'électrode et la poignée, et une position sortie minimum qui définit une seconde distance par rapport à la poignée, et le rapport entre la seconde distance et la première distance est compris entre 0,05 environ et 0,2 environ.

12. Dispositif selon la revendication 1, dans lequel la poignée présente une extrémité proximale et une extrémité distale, l'extrémité distale de la poignée se situe entre l'électrode et l'extrémité proximale de la poignée, et l'électrode est en communication électrique avec un fil de connexion qui s'étend à partir de l'extrémité proximale de la poignée.

13. Dispositif selon la revendication 1, dans lequel la tige allongée présente une extrémité proximale et une extrémité distale, et comprend une surface d'arrêt de la tige qui se situe entre l'extrémité proximale de la tige et l'extrémité distale de la tige, la poignée présente une extrémité proximale et une extrémité distale, et comprend une surface d'arrêt de la poignée qui se situe entre l'extrémité proximale de la poignée et l'extrémité distale de la poignée, de telle sorte que l'engagement de la surface d'arrêt de la tige et de la surface d'arrêt de la poignée, retienne la tige sur la poignée.

14. Dispositif selon la revendication 1, dans lequel la tige peut être détachée de manière sélective de la poignée.
